# EUROPÄISCHE PATENTANMELDUNG

(11) **EP 1 787 631 A1**
(43) Veröffentlichungstag der Anmeldung: **23.05.2007**
(21) Anmeldenummer: 06023418.4
(22) Anmeldetag: 10.11.2006
(51) Int. Cl.: A61K 8/41, A61K 8/49, A61Q 5/10, A61Q 5/06

(54) **Neue Farbstoffvorproduktkombinationen**

(30) Priorität: 17.11.2005 DE 102005055270
(71) Anmelder: HENKEL KOMMANDITGESELLSCHAFT AUF AKTIEN, 40589 Düsseldorf (DE)
(72) Erfinder: Höffkes, Horst, 40595 Düsseldorf (DE); Gross, Wibke, 40549 Düsseldorf (DE); Oberkobusch, Doris, 40591 Düsseldorf (DE); Reichert, Anja, 40629 Düsseldorf (DE); Seiler, Martina, 47228 Duisburg (DE); Knübel, Georg, 40219 Düsseldorf (DE); Kainz, Sabine, 47447 Moers (DE)

(57) **Zusammenfassung**

Gegenstand der vorliegenden Patentanmeldung beansprucht Mittel zur Färbung keratinischer Fasern, insbesondere menschlicher Haare, enthaltend in einem kosmetisch akzeptablen Träger
a) mindestens ein Pyrazolderivat der Formel (I) 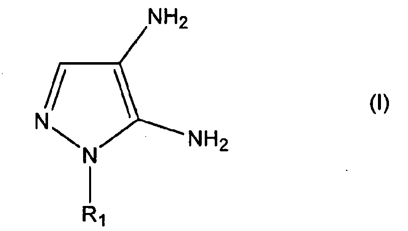 wobei R₁ steht für Wasserstoff, eine geradkettige oder verzweigte C₁₋₆-Alkylgruppe, eine C₁₋₄-Monohydroxyalkylgruppe, eine C₂₋₄-Polyhydroxyalkylgruppe, eine C₁₋₄-Aminoalkylgruppe, eine Phenylgruppe, die gegebenenfalls mit einer Nitrogruppe, einer Trifluormethylgruppe, einer Aminogruppe oder einer C₁₋₄-Alkylaminogruppe substituiert ist oder eine Benzylgruppe, die gegebenenfalls mit einem Halogenatom, einer C₁₋₄-Alkylgruppe, einer C₁₋₄-Alkoxygruppe, einer Methylendioxygruppe oder einer Aminogruppe substituiert ist,
b) mindestens ein substituiertes m-Aminophenoldeirvat der Formel (II) 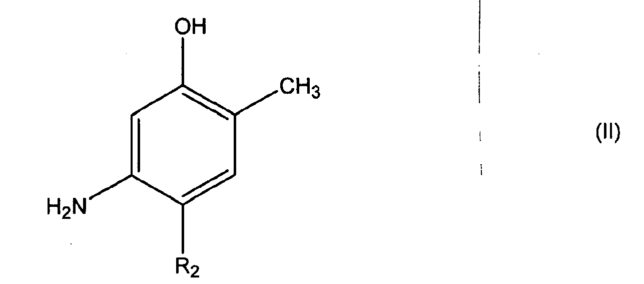 wobei R₂ steht für ein Halogenatom oder eine C₁₋₄-Alkoxygruppe und
c) mindestens einen direktziehenden Farbstoff.

## Beschreibung

Die vorliegende Erfindung betrifft Mittel zur Färbung keratinischer Fasern, die spezielle Pyrazolderivate, spezielle m-Aminophenolderivate und mindestens einen direktziehenden Farbstoff enthalten, sowie ein Verfahren zur Färbung von Haaren mit diesen Mitteln.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Schweiß, Reibung und den Einfluss chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffvorproduktkombinationen, die auch in toxikologischer und dermatologischer Hinsicht unproblematisch sind.

Aufgabe der vorliegenden Erfindung , war es daher, neue Farbstoffvorproduktkombinationenen zu entwickeln, die die an Oxidationsfarbstoffvorproduktkombinationen gestellten Anforderungen erfüllen und Färbungen in einem breiten Farbspektrum mit guten Echtheitseigenschaften ermöglichen.

Überraschenderweise wurde nun gefunden, dass Farbstoffvorproduktkombinationen, die ein spezielles Pyrazolderivat, ein spezielles m-Aminophenolderivat und ein mindestens einen direktziehenden Farbstoff enthalten, den an derartige Kombinationen gestellten Anforderungen in einem hohen Maße genügen. Die erfindungsgemäßen Farbstoff-(vorprodukt)kombinationen ermöglichen Haarfärbungen mit hoher Farbbrillanz, insbesondere im Rot- bis Rotorangebereich.

Ein erster Gegenstand der vorliegenden Erfindung sind daher Mittel zur Färbung keratinischer Fasern, insbesondere menschlicher Haare, enthaltend in einem kosmetisch akzeptablen Träger
a) mindestens ein Pyrazolderivat der Formel (I) 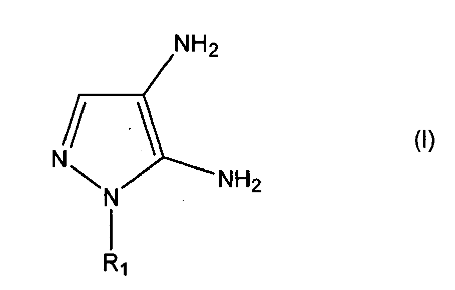 wobei R₁ steht für Wasserstoff, eine geradkettige oder verzweigte C₁₋₆-Alkylgruppe, eine C₁₋₄-Monohydroxyalkylgruppe, eine C₂₋₄-Polyhydroxyalkylgruppe, eine C₁₋₄-Aminoalkylgruppe, eine Phenylgruppe, die gegebenenfalls mit einer Nitrogruppe, einer Trifluormethylgruppe, einer Aminogruppe oder einer C₁₋₄-Alkylaminogruppe substituiert ist oder eine Benzylgruppe, die gegebenenfalls mit einem Halogenatom, einer C₁₋₄-Alkylgruppe, einer C₁₋₄-Alkoxygruppe, einer Methylendioxygruppe oder einer Aminogruppe substituiert ist,
b) mindestens ein substituiertes m-Aminophenolderivat der Formel (II) 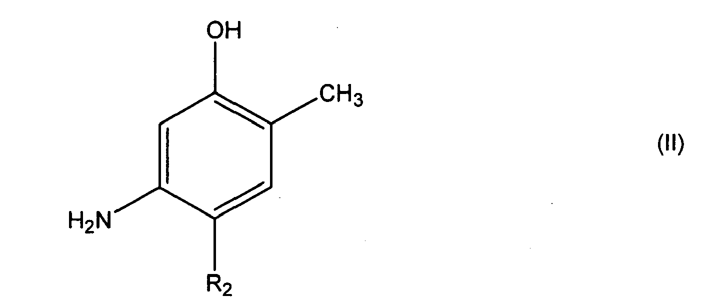 wobei R₂ steht für ein Halogenatom oder eine C₁₋₄-Alkoxygruppe und
c) mindestens einen direktziehenden Farbstoff.

Unter keratinischen Fasern sind dabei erfindungsgemäß Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Da es sich bei den erfindungsgemäßen Farbstoffvorprodukten um Amino-Verbindungen handelt, lassen sich aus diesen in üblicher Weise die bekannten Säureadditionssalze herstellen. Alle Aussagen dieser Schrift und demgemäß der beanspruchte Schutzbereich beziehen sich daher sowohl auf die in freier Form vorliegenden Verbindungen als auch auf deren wasserlösliche, physiologisch verträglichen Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt.

Beispiele für die als Substituenten in den erfindungsgemäßen Verbindungen genannten C₁- bis C₆-Alkylgruppen sind die Gruppen Methyl, Ethyl, Propyl, Isopropyl, n-Butyl, 2-Methylpropyl, n-Pentyl, 2-Methylbutyl, 2,2-Dimethylpropyl, n-Hexyl, 2-Methylpentyl, 3-Methylpentyl, 2,3-Dimethylbutyl sowie 2,2-Dimethylbutyl. Ethyl und Methyl sind bevorzugte Alkylgruppen. Bevorzugte C₁- bis C₄-Alkoxygruppen sind die Gruppen Methoxy und Ethoxy. Weiterhin können als bevorzugte Beispiele für eine C₁- bis C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die übrigen verwendeten Bezeichnungen leiten sich von den hier gegebenen Definitionen ab.

Im Rahmen der vorliegenden Erfindung hat es sich als besonders bevorzugt erwiesen, wenn das Pyrazolderivat der Formel (I) ausgewählt ist aus 4,5-Diamino-1-(4-chlorbenzyl)pyrazol oder 4,5-Diamino-1-(2-hydroxyethyl)-pyrazol sowie beliebigen Gemischen der voranstehenden. Ein erfindungsgemäß ganz besonders bevorzugtes Pyrazolderivat ist 4,5-Diamino-1-(2-hydroxyethyl)-pyrazol.

Im Rahmen der vorliegenden Erfindung hat es sich weiterhin als besonders bevorzugt erwiesen, wenn das m-Aminophenolderivat der Formel (II) ausgewählt ist aus 5-Amino-4-fluor-2-methylphenol, 5-Amino-4-chlor-2-methylphenol oder 5-Amino-4-methoxy-2-methylphenol. Ein erfindungsgemäß ganz besonders bevorzugtes m-Aminophenolderivat ist 5-Amino-4-chlor-2-methylphenol.

Als direktziehende Farbstoffe kommen prinzipiell alle in der Haarfärberei bekannten Farbstoffe in Frage, die direkt auf die Fasern aufziehen. Dabei sind sowohl kationische, nichtionische als auch anionische Farbstoffe geeignet. Als erfindungsgemäß besonders bevorzugt haben sich die nichtionischen Farbstoffe erweisen.

Im Rahmen der vorliegenden Erfindung haben sich jedoch die folgenden Farbstoffe als besonders bevorzugt erwiesen: 1-Amino-4-(methylamino)anthrachinon, 1,4-Diamino-2-nitrobenzol, 1-(2-Hydroxyethyl)amino-2-(2-hydroxyethyl)methoxy-4-nitrobenzol, 1-(2-Hydroxyethyl)amino-2-nitrobenzol, 4'-Hydroxy-2-nitrodiphenylamin, 4-Amino-1-(2-hydroxyethyl)amino-2-nitrobenzol, 4-Amino-3-nitrophenol, 4-(2-Hydroxyethyl)amino-3-nitrophenol, 1-(2-Ureidoethyl)amino-4-nitrobenzol, 2-(2'-Hydroxyethyl)amino-1-hydroxy-4,6-dinitrobenzol, 1-(2-Hydroxyethyl)amino-4-methyl-2-nitrobenzol, 4-(2-Hydroxyethyl)amino-1-chlor-3-nitrobenzol, 4-Ethylamino-3nitrobenzoesäure, 2-Chlor-6-ethylamino-4-nitrophenol, 2-Amino-6-chlor-4-nitrophenol, 4-(3-Hydroxypropyl)amino-3-nitrophenol, 4-[(4-Methyl-2-sulfophenyl(amino]-1-hydroxyanthrachinon, 1,4-Diaminoanthrachinon, 2,2-[[4-(4-aminophenylazo)phenyl]imino]bis-ethanol, 1-Propen-(4-amino-2-nitrophenyl)amin, 2-Amino-4,6-dinitrophenol, 6-Ethylamino-2-chlor-4-nitrophenol, 2-Hydroxyethyl-pikraminsäure, 3-((Pyridin-3-yl)azo)pyridin, 4-Nitrophenylaminoethyl-harnstoff, 1-Amino-4-bis-(2-hydroxyethyl)amino-2-nitrobenzol, 1,4-Bis-(2-hydroxyethyl)amino-2-nitrobenzol, 2-Amino-1-(2-hydroxyethyl)amino-4-nitrobenzol, 1-(2-Hydroxyethyl)amino-4-bis-(2-hydroxyethyl) amino-2-nitrobenzol, 1-(2-Hydroxyethyl)amino-4-(ethyl, 2-hydroxyethyl)amino-2-nitrobenzol sowie die unter den CTFA-Bezeichnungen Basic Yellow 87, Basic Orange 31 und Basic Red 51 bekannten Farbstoffe der Formeln 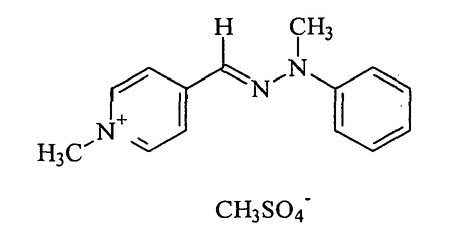 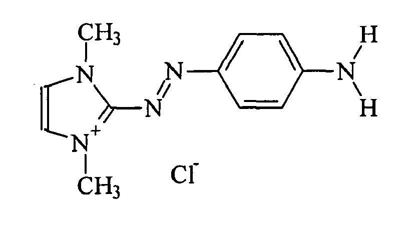 und

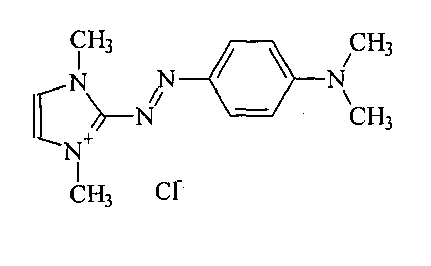

Ganz besonders bevorzugte direktziehende Farbstoffe sind im Sinne der vorliegenden Erfindung 1,4-Diamino-2-nitrobenzol, 4'-Hydroxy-2-nitrodiphenylamin, 4-Amino-3-nitrophenol, 2-(2'-Hydroxyethyl)amino-1-hydroxy-4,6-dinitrobenzol, 1-(2-Hydroxyethyl)amino-4-methyl-2-nitrobenzol, 4-Ethylamino-3-nitrobenzoesäure, 2-Chlor-6-ethylamino-4-nitrophenol, 6-Ethylamino-2-chlor-4-nitrophenol, 2-Hydroxyethyl-pikraminsäure und 1-Propen-4-(amino-2-nitropheny)amin.

Bevorzugte direktziehende Farbstoffe sind weiterhin die unter den internationalen Bezeichnungen bzw. Handelsnamen HC Yellow 6, Acid Yellow 1, Acid Yellow 10, Acid Yellow 23, Acid Yellow 36, Disperse Orange 3, Acid Orange 7, HC Red 1, HC Red 10, HC Red 11, Acid Red 33, Acid Red 52, Pigment Red 57:1, Acid Blue 7, Acid Green 50, Acid Black 1 und Acid Black 52 bekannten Verbindungen sowie 2-Amino-4-nitrophenol, 1-Amino-4-(2'-hydroxyethyl)-amino-5-chlor-2-nitrobenzol, 4-Amino-2-nitrodiphenylamin-2'-carbonsäure, 6-Nitro-1,2,3,4-tetrahydrochinoxalin, 2-Hydroxy-1,4-naphthochinon sowie Pikraminsäure und deren Salze.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quatemäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

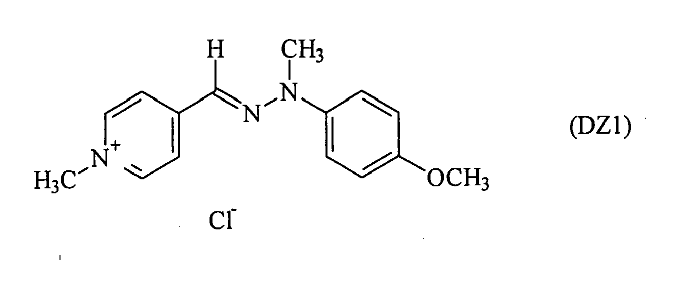

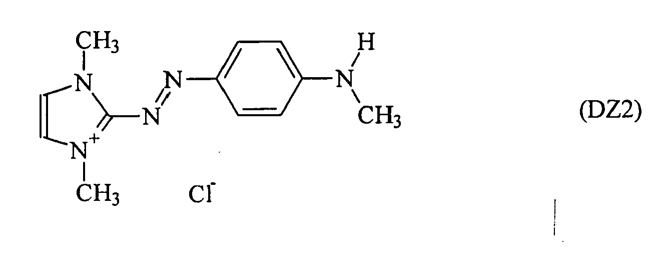

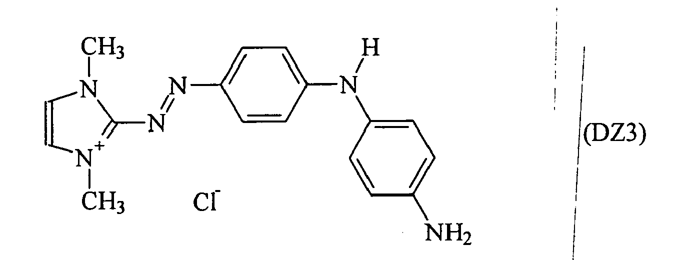

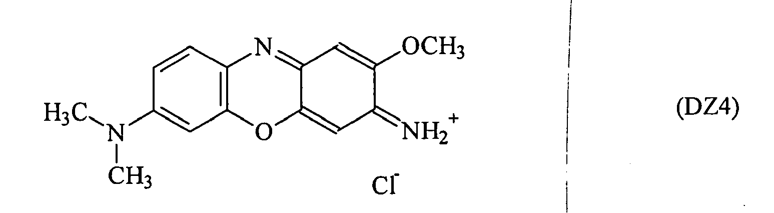

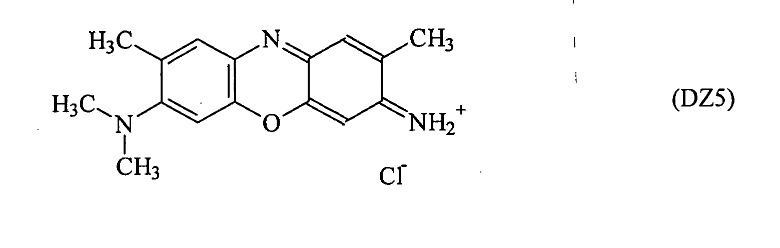

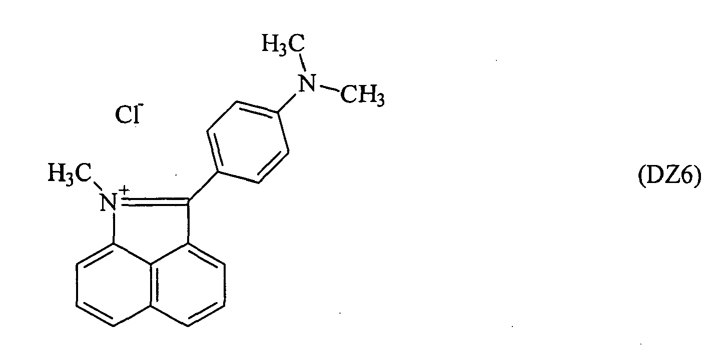

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin hat es sich im Rahmen der vorliegenden Erfindung als vorteilhaft erwiesen, wenn die Mittel als Alkalisierungsmittel ein organisches Amin enthalten.

Erfindungsgemäß bevorzugte organische Amine sind Monoethanolamin, Diethanolamin, Triethanolamin, 2-Amino-2-methyl-1-propanol, 2-Amino-2-methyl-1,3-propandiol, Monoisopropanolamin und Tetrakis(2-hydroxyisopropyl)-ethylendiamin.

Besonders bevorzugt ist es erfindungsgemäß, wenn als Alkalisierungsmittel Monoethanolamin, 2-Amino-2-methyl-1-propanol und 2-Amino-2-methyl-1,3-propandiol, eingesetzt wird. Monoethanol ist ein erfindungsgemäß ganz besonders bevorzugtes Alkalisierungsmittel.

Im Rahmen dieser Ausführungsform hat es sich als besonders bevorzugt erwiesen, wenn das Mittel einen direktziehenden Farbstoff, ausgewählt aus 1-Amino-4-bis-(2-hydroxyethyl)amino-2-nitrobenzol, 1,4-Bis-(2-hydroxyethyl)amino-2-nitrobenzol, 2-Amino-1-(2-hydroxyethyl)amino-4-nitrobenzol, 1-(2-Hydroxyethyl)amino-4-bis-(2-hydroxyethyl) amino-2-nitrobenzol, 1-(2-Hydroxyethyl)amino-2-(2-hydroxyethyl)methoxy-4-nitrobenzol, 1-(2-Hydroxyethyl)amino-2-nitrobenzol, 4-Amino-1-(2-hydroxyethyl)amino-2-nitrobenzol, 4-(2-Hydroxyethyl)amino-3-nitrophenol, 1-(2-Hydroxyethyl)amino-4-(ethyl, 2-hydroxyethyl)amino-2-nitrobenzol, 4-(2-Hydroxyethyl)amino-1-chlor-3-nitrobenzol und 4-(3-Hydroxypropyl)amino-3-nitrophenol enthält.

Im Rahmen einer weiteren bevorzugten Ausführungsform hat es sich als bevorzugt erwiesen, wenn die Mittel der vorliegenden Erfindung frei von Reduktionsmitteln formuliert werden. Ausgenommen von dieser Regelung sind erfindungsgemäß die Farbstoff(vorprodukt)e, insbesondere die Entwicklerkomponenten, die selbst als Reduktionsmittel wirken können.

Weiterhin hat es sich als vorteilhaft erwiesen, wenn die erfindungsgemäßen Mittel mindestens eine weitere Entwicklerkomponente enthalten.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 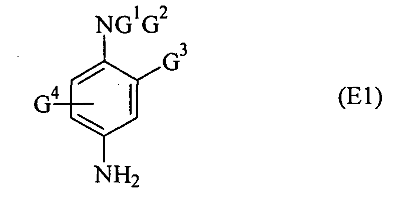 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄ -Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluyiendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 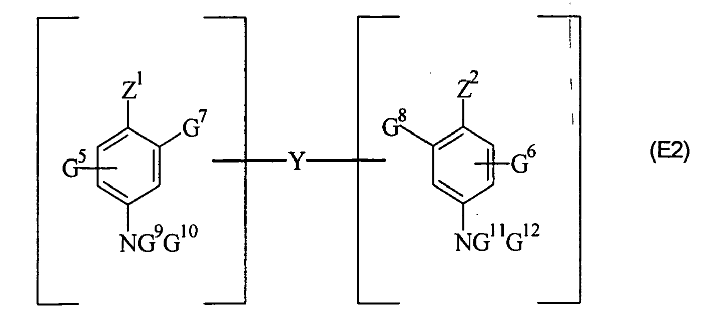 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
   mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere:
N,N'-Bis-(β-hydroxyethyl)-N,N'-bis-(4'-aminopheny)-'1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)tetramethylendiamin, N,N'-Bis-(4-methyl-aminophenyl)-tetramethylendiamin, N.N'-Diethyl-N, N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 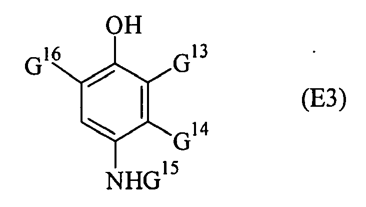 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-PyrimidinDerivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazolopyrimidin-Derivate sind insbesondere die Derivate des Pyrazolo[1,5-a]pyrimidin der folgenden Formel (E4) und dessen tautomeren Formen, sofern ein tautomeres Gleichgewicht besteht: 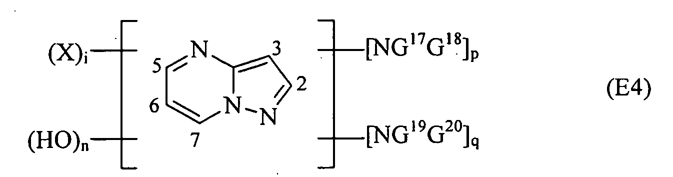 wobei:
- G¹⁷ , G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁ bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷ G¹⁸ (oder NG¹⁹G ²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazolo[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

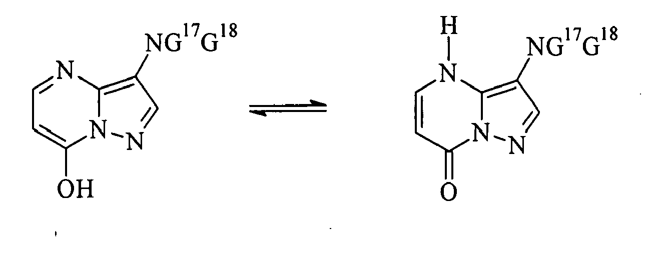

Unter den Pyrazolo[1,5-a]-pyrimidinen der obenstehenden Formel (E4) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hiydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7 -diamin;
- 3-Amino-7-dimethylamino-2,5-dimethylpyrazölo[1,5-a]pyrimidin;
   sowie ihre physiologisch verträglichen Salze und ihre tautomeren Formen, wenn ein tautomers Gleichgewicht vorhanden ist.
   Die Pyrazolo[1,5-a]pyrimidine der obenstehenden Formel (E4) können wie in der Literatur beschrieben durch Zyklisierung ausgehend von einem Aminopyrazol oder von Hydrazin hergestellt werden.
   In einer weiteren bevorzugten Ausführungsform enthalten die erfindungsgemäßen Färbemittel mindestens eine Kupplerkomponente.
   Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.
   Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethyl phenyl}-amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
   sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Neben den erfindungsgemäß genannten Verbindungen enthalten die Färbemittel der vorliegenden Anmeldung vorzugsweise mindestens eine weitere Entwicklerkomponente, ausgewählt aus p-Phenylendiamin, p-Toluylendiamin, 2-(2-Hydroxyethyl)-p-phenylendiamin, 4-Aminophenol, 3-Methyl-4-aminophenol, Bis-(2-hydroxy-5-aminophenyl)methan und 2,4,5,6-Tetraaminopyrimidin, und/oder mindestens eine weitere Kupplerkomponente, ausgewählt aus 1-Naphthol, 1,5- Dihydroxynaphthalin, 2,7-Dihydroxynaphthalin, 3-Aminophenol, 5-Amino-2-methylphenol, 5-(2-Hydroxyethyl)amino-2-methylphenol, 2-Chlor-6-methyl-3-aminophenol, 2,4-Dichlor-3-aminophenol, 2-Amino-3-hydroxypyridin, Resorcin, 2-Methylresorcin, 2,4-Diaminophenoxyethanol, 1,3-Bis-(2,4-diaminophenoxy)-propan sowie 1-Methoxy-2-amino-4-(2'-hydroxyethylamino)benzol.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

In einer weiteren Ausführungsform der vorliegenden Erfindung können die Färbemittel mindestens eine Vorstufe eines naturanalogen Farbstoffs enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IVa), 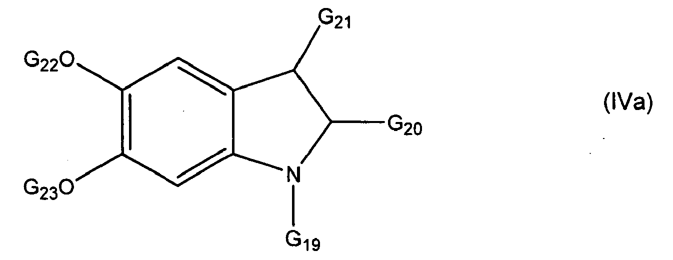 in der unabhängig voneinander
- G₁₉ steht für Wasserstoff, eine C₁-C₄-Alkylgruppe oder eine C₁-C₄-Hydroxy-alkylgruppe,
- G₂₀ steht für Wasserstoff oder eine -COOH-Gruppe, wobei die -COOH-Gruppe auch als Salz mit einem physiologisch verträglichen Kation vorliegen kann,
- G₂₁ steht für Wasserstoff oder eine C₁-C₄-Alkylgruppe,
- G₂₂ steht für Wasserstoff, eine C₁-C₄-Alkylgruppe oder eine Gruppe -CO-G₂₄, in der G₂₄ steht für eine C₁-C₄-Alkylgruppe, und
- G₂₃ steht für eine der unter G₂₂ genannten Gruppen,
   sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbon-säure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IVb), 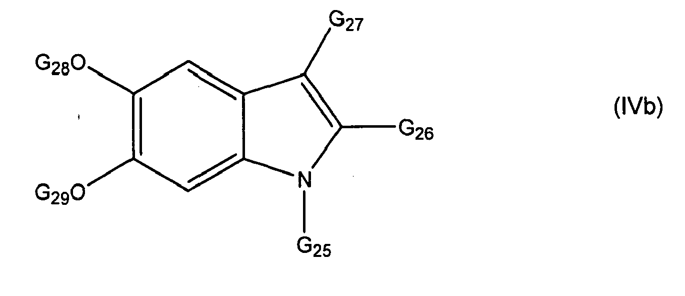 in der unabhängig voneinander
- G₂₅ steht für Wasserstoff, eine C₁-C₄-Alkylgruppe oder eine C₁-C₄-Hydroxyalkylgruppe,
- G₂₆ steht für Wasserstoff oder eine -COOH-Gruppe, wobei die -COOH-Gruppe auch als Salz mit einem physiologisch verträglichen Kation vorliegen kann,
- G₂₇ steht für Wasserstoff oder eine C₁-C₄-Alkylgruppe,
- G₂₈ steht für Wasserstoff, eine C₁-C₄-Alkylgruppe oder eine Gruppe -CO-G₃₀, in der G₃₀ steht für eine C₁-C₄-Alkylgruppe, und
- G₂₉ steht für eine der unter G₂₈ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹ O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im Wesentlichen aus C₈- und C₁₀-Alkylgruppen,
- im Wesentlichen aus C₁₂- und C₁₄-Alkylgruppen,
- im Wesentlichen aus C₈- bis C₁₆-Alkylgruppen oder
- im Wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der lNCl-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Grupp enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den lNCl-Bezeichnungen Quaternium-27 und Quaternium-83 bekannten lmidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quatemium-80).

Ein Beispiel für ein als kationisches Tensid einsetzbares quatemäres Zuckerderivat stellt das Handelsprodukt Glucquat^{®}100 dar, gemäß lNCl-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
   enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Farbstoff(vorprodukt)e bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol beziehungsweise Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Anmeldung ist Verfahren zur Färbung keratinischer Fasern, bei dem ein erfindungsgemäßes Haarfärbemittel auf die Fasern aufgetragen wird und nach einer Einwirkzeit wieder abgespült wird.

Ein dritter Gegenstand der vorliegenden Anmeldung ist ein Verfahren zur Färbung keratinischer Fasern, bei dem eine Anwendungszubereitung hergestellt wird aus einer ersten Komponente (A), enthaltend mindestens ein Farbstoffvorprodukt, einer zweiten Komponenten (B), enthaltend mindestens einen direktziehenden Farbstoff, und einer dritten Komponente (C), enthaltend eine Oxidationsmittelzubereitung, diese Anwendungszubereitung auf die Fasern aufgetragen wird und nach einer Einwirkzeit wieder abgespült wird,
wobei die Komponente (A) mindestens ein Pyrazolderivat der Formel (I) 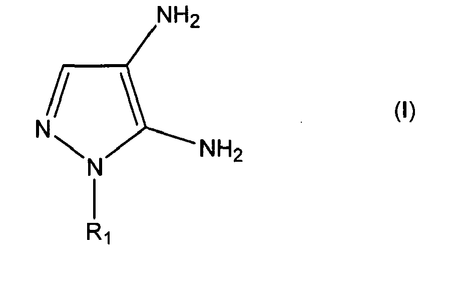 wobei R₁ steht für Wasserstoff, eine geradkettige oder verzweigte C₁₋₆-Alkylgruppe, eine C₁₋₄-Monohydroxyalkylgruppe, eine C₂₋₄-Polyhydroxyalkylgruppe, eine C₁₋₄-Aminoalkylgruppe, eine Phenylgruppe, die gegebenenfalls mit einer Nitrogruppe, einer Trifluormethylgruppe, einer Aminogruppe oder einer C₁₋₄-Alkylaminogruppe substituiert ist oder eine Benzylgruppe, die gegebenenfalls mit einem Halogenatom, einer C₁₋₄-Alkylgruppe, einer C₁₋₄-Alkoxygruppe, einer Methylendioxygruppe oder einer Aminogruppe substituiert ist, und mindestens ein substituiertes m-Aminophenolderivat der Formel (II) 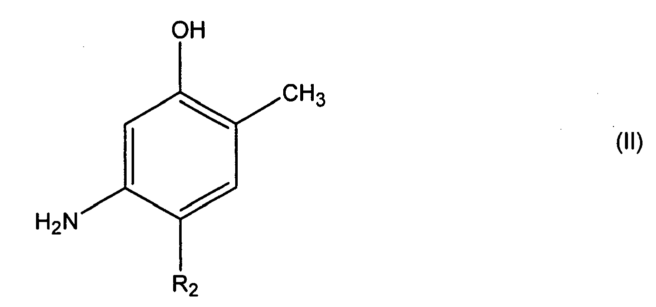 wobei R₂ steht für ein Halogenatom oder eine C₁₋₄-Alkoxygruppe, enthält.

Insbesondere im Rahmen dieser Ausführungsform sind hinsichtlich der Wahl des einzusetzenden Farbstoffes keinerlei Einschränkungen vorhanden.

Es können in einer bevorzugten Ausführungsform alle im Rahmen dieser Anmeldung konkret genannten direktziehenden Farbstoffe zum Einsatz kommen.

Auch hinsichtlich der Konfektionierungsformen der drei Zubereitungen gemäß dieses Gegenstandes der vorliegenden Erfindung sollen keine Einschränkungen gelten. So gelten die oben getroffenen Aussagen über die verschiedenen Konfektionierungsformen analog auch für die drei Teilzubereitungen gemäß diesem Gegenstand der vorliegenden Anmeldung.

Allerdings hat es sich als erfindungsgemäß besonders bevorzugt erwiesen, wenn die die Komponente (B), enthaltend mindestens einen direktziehenden Farbstoff, in Form eine O/W-Emulsion oder einer wasserfreien Paste formuliert wird.

### Ausführungsbeispiele:

### 1 Versuchsdurchführung

Die in den Beispielen verwendeten Mengenangaben verstehen sich, wenn nichts anderes vermerkt ist, in Gewichtsprozent. Es wurden die folgenden Färbecremes hergestellt:

| Rohstoff | 1 | 2 | 3 |
|---|---|---|---|
| Stenol^{®} 1618 | 6,0 | 6,0 | 6,0 |
| Kokoslorol^{®} | 6,0 | 6,0 | 6,0 |
| Eumulgin^{®} B1 | 3,0 | 3,0 | 3,0 |
| Eumulgin^{®} B 2 | 3,0 | 3,0 | 3,0 |
| Eumulgin^{®} HRE 40 | 1,0 | 1,0 | 1,0 |
| Polydiol^{®} 400 | 5,0 | 5,0 | 5,0 |
| Aminoxyd^{®} WS 35 | 1,0 | 1,0 | 1,0 |
| EDTA-Dinatriumsalz | 0,1 | 0,1 | 0,1 |
| Natrosol^{®} 250 HHR | 1,0 | 1,0 | 1,0 |
| Ammoniumsulfat | 0,4 | 0,4 | 0,4 |
| Ascorbinsäure | 0,1 | 0,1 | 0,1 |
| Hydroxyethandiphosphonsäure | 0,2 | 0,2 | 0,2 |
| Parfümöl | 0,3 | 0,3 | 0,3 |
| Arginin | 2,0 | 2,0 | 2,0 |
| 4,5-Diamino-1-(2-hydroxyethyl)pyrazolsulfat | 0,60 | 0,84 | 0,72 |
| 5-Amino-4-chlor-2-methylphenol | 0,31 | 0,47 | 0,24 |
| 1-Naphthol | - | 0,07 | - |
| 2,6-Dihydroxy-3,4-dimethylpyridin | 0,07 | - | 0,21 |
| 4'-Hydroxy-2-nitrodiphenylamin | 0,02 | - | - |
| 4-Amino-3-nitrophenol | 0,02 | - | - |
| 1-(2-Hydroxyethyl)amino-2-(2-hydroxyethyl)methoxy-4-nitrobenzol | - | - | 0,05 |
| 4-(2-Hydroxyethyl)amino-3-nitrophenol | - | 0,15 | - |
| 1-(2-Hydroxyethyl)amino-4-methyl-2-nitrobenzol | 0,02 | - | 0,1 |
| 2-Amino-6-chlor-4-nitrophenol | 0,05 | 0,02 | 0,05 |
| 2-Chlor-6-ethylamino-4-nitrophenol | - | 0,1 | - |
| Monoethanolamin | ad pH 10 | ad pH 10 | ad pH 10 |
| Wasser | ad 100 | ad 100 | ad 100 |
| Ausfärbung | Orangerot | Neutrales Rot | Rot-orange |

Für die Ausfärbungen wurde die Färbecreme im Verhältnis 1:1 mit der folgenden Oxidationsmittelzubereitung vermischt:

| | |
|---|---|
| Dipicolinsäure | 0,1 Gew.-% |
| Natriumpyrophosphat | 0,03 Gew.-% |
| Turpinal^{®} SL | 1,50 Gew.-% |
| Texapon^{®} N28 | 2,00 Gew.-% |
| Acrysol^{®} 33 | 12,00 Gew.-% |
| Wasserstoffperoxid, 50 %ig | 12,00 Gew.-% |
| Ammoniak, 25%ig | 0,62 Gew.-% |
| Wasser | ad 100 Gew.-% |

In jede der so erhaltenen Mischungen wurde eine Haarsträhne (80 % ergraut; 330 mg bis 370 mg schwer) gegeben. Anschließend wurden die Mischungen und die Haarsträhnen auf jeweils ein Uhrglas gegeben und die Haarsträhnen in die Färbecremes gut eingebettet. Nach 30 Minuten (±1 Minute) Einwirkzeit bei Raumtemperatur wurden die Haarsträhnen entnommen und mit einer wässrigen Texapon^{®} EVR-Lösung so oft gewaschen, bis der Farbüberschuss entfernt war. Die Haarsträhnen wurden an der Luft getrocknet und ihr Farbton unter der Tageslichtlampe (Farbprüfgerät HE240A) bestimmt und notiert.

### 2 Verzeichnis der eingesetzten Handelsprodukte

| | |
|---|---|
| Acrysol^{®} 33 | Acrylpolymer (ca. 28% Festkörper in Wasser; INCI-Bezeichnung: Acrylates Copolymer) |
| Aminoxyd^{®} WS 35 | N,N-Dimethyl-N(C₈₋₁₈-kokosacylamidopropyl)amin-N-oxid (ca. 35% Aktivsubstanz in Wasser; lNCl-Bezeichnung: Cocamidopropylamine Oxide) (Goldschmidt) |
| Eumulgin^{®} B 1 | Cetylstearylalkohol mit ca. 12-EO-Einheiten (INCI-Bezeichnung: Ceteareth-12) (Cognis) |
| Eumulgin^{®} B2 | Cetylstearylalkohol mit ca. 20 EO-Einheiten (INCI-Bezeichnung: Ceteareth-20) (Cognis) |
| Eumulgin^{®} HRE 40 | gehärtetes Rizinusöl mit ca. 40-EO-Einheiten (INCI-Bezeichnung: Peg-40 Hydrogenated Castor Oil) (Cognis) |
| Kokoslorol^{®} | C12-18-Fettalkohol (INCI-Bezeichnung: Coconut Alcohol) (Cognis) |
| Natrosol^{®} 250 HHR | Hydroxyethylcellulose (INCI-Bezeichnung: Hydroxyethylcellulose) (Hercules) |
| Polydiol^{®} 400 | Polyethylenglykol (lNCl-Bezeichung: PEG-8) (Cognis) |
| Stenol^{®} 1618 | C₁₆-₁₈-Fettalkohol (INCI-Bezeichnung : Cetearyl Alcohol) (Cognis) |
| Texapon^{®} EVR | Laurylethersulfat-Natrium-Salz mit speziellen Zusätzen (ca. 34 bis 37% Aktivsubstanzgehalt; INCI-Bezeichnung: Sodium Lauryl Sulfate, Sodium Laureth Sulfate, Lauramide MIPA, Cocamide MEA, Glycol Stearate, Laureth-10) (Cognis) |
| Texapon^{®} N28 | Laurylethersulfat-Natrium-Salz (mind. 26,5 % Aktivsubstanzgehalt; INCI-Bezeichnung: Sodium Laureth Sulfate) (Cognis) |
| Turpinal^{®} SL | 1-Hydroxyethan-1,1-diphosphonsäure (ca. 58 - 61% Aktivsubstanzgehalt; INCI-Bezeichnung: Etidronic Acid, Aqua (Water) (Solutia) |

## Patentansprüche

1. Mittel zur Färbung keratinischer Fasern, insbesondere menschlicher Haare, enthaltend in einem kosmetisch akzeptablen Träger
a) mindestens ein Pyrazolderivat der Formel (I) 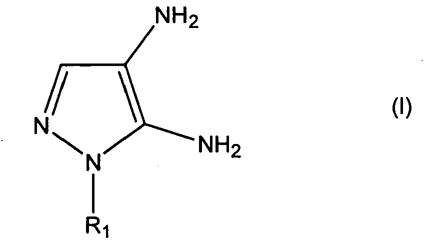 wobei R₁ steht für Wasserstoff, eine geradkettige oder verzweigte C₁₋₆-Alkylgruppe, eine C₁₋₄-Monohydroxyalkylgruppe, eine C₂₋₄-Polyhydroxyalkylgruppe, eine C₁₋₄-Aminoalkylgruppe, eine Phenylgruppe, die gegebenenfalls mit einer Nitrogruppe, einer Trifluormethylgruppe, einer Aminogruppe oder einer C₁₋₄-Alkylaminogruppe substituiert ist oder eine Benzylgruppe, die gegebenenfalls mit einem Halogenatom, einer C₁₋₄-Alkylgruppe, einer C₁₋₄-Alkoxygruppe, einer Methylendioxygruppe oder einer Aminogruppe substituiert ist,
b) mindestens ein substituiertes m-Aminophenolderivat der Formel (II) 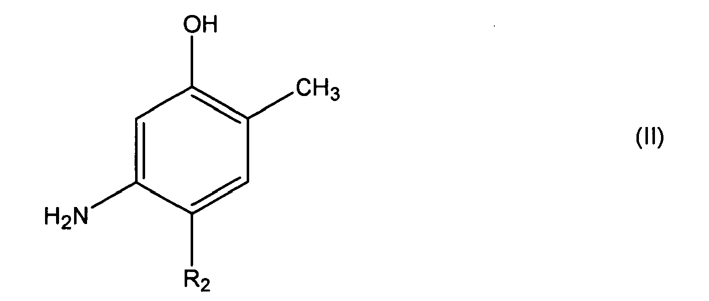 wobei R₂ steht für ein Halogenatom oder eine C₁₋₄-Alkoxygruppe und
c) mindestens einen direktziehenden Farbstoff.

2. Mittel nach Anspruch 1, **dadurch gekennzeichnet, dass** es als Pyrazolderivat 4,5-Diamino-1-(4-chlorbenzyl)pyrazol oder 4,5-Diamino-1-(2-hydroxyethyl)-pyrazol sowie beliebige Gemischen der voranstehenden enthält.

3. Mittel nach Anspruch 2, **dadurch gekennzeichnet, dass** es als Pyrazolderivat 4,5 Diamino-1-(2-hydroxyethyl)-pyrazol enthält.

4. Mittel nach einem der Ansprüche 1 bis 3, **dadurch gekennzeichnet, dass** es als m-Aminophenolderivat der Formel (II) 5-Amino-4-fluor-2-methylphenol, 5-Amino-4-chlor-2-methylphenol oder 5-Amino-4-methoxy-2-methylphenol enthält.

5. Mittel nach Anspruch 4, **dadurch gekennzeichnet, dass** es als m-Aminophenolderivat der Formel (II) 5-Amino-4-chlor-2-methylphenol enthält.

6. Mittel nach einem der Ansprüche 1 bis 5, **dadurch gekennzeichnet, dass** es einen direktziehenden Farbstoff, ausgewählt aus 1-Amino-4-(methylamino)anthrachinon, 1,4-Diamino-2-nitrobenzol, 1-(2-Hydroxyethyl)amino-2-(2-hydroxyethyl)methoxy-4-nitrobenzol, 1-(2-Hydroxyethyl)amino-2-nitrobenzol, 4'-Hydroxy-2-nitrodiphenylamin, 4-Amino-1-(2-hydroxyethyl)amino-2-nitrobenzol, 4-Amino-3-nitrophenol, 4-(2-Hydroxyethyl)amino-3-nitrophenol, 1-(2-Ureidoethyl)amino-4-nitrobenzol, 2-(2'-Hydroxyethyl)amino-1-hydroxy-4,6-dinitrobenzol, 1-(2-Hydroxyethyl)amino-4-methyl-2-nitrobenzol, 4-(2-Hydroxyethyl)amino-1-chlor-3-nitrobenzol, 4-Ethylamino-3nitrobenzoesäure, 2-Chlor-6-ethylamino-4-nitrophenol, 2-Amino-6-chlor-4-nitrophenol, 4-(3-Hydroxypropyl)amino-3 nitrophenol, 4-[(4-Methyl-2-sulfophenyl(amino]-1-hydroxyanthrachinon, 1,4-Diaminoanthrachinon, 2,2-[[4-(4-aminophenylazo)phenyl]imino]bis-ethanol, 1-Propen-(4-amino-2-nitrophenyl)amin, 2-Amino-4,6-dinitrophenol, 6-Ethylamino-2-chlor-4-nitrophenol, 2-Hydroxyethyl-pikraminsäure, 3-((Pyridin-3-yl)azo)pyridin, 4-Nitrophenylaminoethy)-harnstoff, 1-Amino-4-bis-(2-hydroxyethyl)amino-2-nitrobenzol, 1,4-Bis-(2-hydroxyethyl)amino-2-nitrobenzol, 2-Amino-1-(2-hydroxyethyl)amino-4-nitrobenzol, 1-(2-Hydroxyethyl)amino-4-bis-(2-hydroxyethyl) amino-2-nitrobenzol, 1-(2-Hydroxyethyl)amino-4-(ethyl, 2-hydroxyethy)amino-2-nitrobenzol sowie den unter den CTFA-Bezeichnungen Basic Yellow 87, Basic Orange 31 und Basic Red 51 bekannten kationischen Farbstoffen enthält.

7. Mittel nach Anspruch 6, **dadurch gekennzeichnet, dass** es einen direktziehenden Farbstoff, ausgewählt aus 1,4-Diamino-2-nitrobenzol, 4'-Hydroxy-2-nitrodiphenylamin, 4-Amino-3-nitrophenol, 2-(2'-Hydroxyethyl)amino=1-hydroxy-4,6-dinitrobenzol, 1-(2-Hydroxyethyl)amino-4-methyl-2-nitrobenzol, 4-Ethylamino-3-nitrobenzoesäure, 2-Chlor-6-ethylamino-4-nitrophenol, 6-Ethylamino-2-chlor-4-nitrophenol, 2-Hydroxyethyl-pikraminsäure und 1-Propen-4-(amino-2-nitrophenyl) amin enthält.

8. Mittel nach einem der Ansprüche 1 bis 7, **dadurch gekennzeichnet, dass** es als Alkalisierungsmittel ein organisches Amin enthält.

9. Mittel nach Anspruch 8, **dadurch gekennzeichnet, dass** es als Alkalisierungsmittel Monoethanolamin enthält.

10. Mittel nach Anspruch 9, **dadurch gekennzeichnet, dass** es einen direktziehenden Farbstoff, ausgewählt aus 1-Amino-4-bis-(2-hydroxyethyl)amino-2-nitrobenzol, 1,4-Bis-(2-hydroxyethyl)amino-2-nitrobenzol, 2-Amino-1-(2-hydroxyethyl)amino-4-nitrobenzol, 1-(2-Hydroxyethyl)amino-4-bis-(2-hydroxyethyl)amino-2-nitrobenzol, 1-(2-Hydroxyethyl)amino-2-(2-hydroxyethyl)methoxy-4-nitrobenzol, 1-(2-Hydroxyethyl)amino-2-nitrobenzol, 4-Amino-1-(2-hydroxyethyl)amino-2-nitrobenzol, 4-(2-Hydroxyethyl)amino-3-nitrophenol, 1-(2-Hydroxyethyl)amino-4-(ethyl, 2-hydroxyethyl)amino-2-nitrobenzol, 4-(2-Hydroxyethyl)amino-1-chlor-3-nitrobenzol und 4-(3-Hydroxypropyl)amino-3-nitrophenol, enthält.

11. Mittel nach einem der Ansprüche 1 bis 10, **dadurch gekennzeichnet, dass** es neben den Farbstoffvorprodukten kein Reduktionsmittel enthält.

12. Mittel nach einem der Ansprüche 1 bis 11; **dadurch gekennzeichnet, dass** es mindestens eine weitere Entwicklerkomponente enthält.

13. Mittel nach einem der Ansprüche 1 bis 12, **dadurch gekennzeichnet, dass** es mindestens eine weitere Kupplerkomponente enthält.

14. Verfahren zur Färbung keratinischer Fasern, bei dem ein Mittel nach einem der Ansprüche 1 bis 13 auf die Fasern aufgetragen wird und nach einer Einwirkzeit wieder abgespült wird.

15. Verfahren zur Färbung keratinischer Fasern, bei dem eine Anwendungszubereitung hergestellt wird aus einer ersten Komponente (A), enthaltend mindestens ein Farbstoffvorprodukt, einer zweiten Komponenten (B), enthaltend mindestens einen direktziehenden Farbstoff, und einer dritten Komponente (C), enthaltend eine Oxidationsmittelzubereitung, diese Anwendungszubereitung auf die Fasern aufgetragen wird und nach einer Einwirkzeit wieder abgespült wird,
wobei die Komponente (A) mindestens ein Pyrazolderivat der Formel (I) 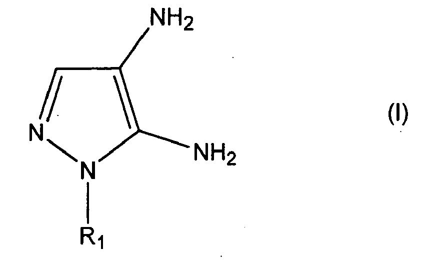 wobei R₁ steht für Wasserstoff, eine geradkettige oder verzweigte C₁₋₆-Alkylgruppe, eine C₁₋₄-Monohydroxyalkylgruppe, eine C₂₋₄-Polyhydroxyalkylgruppe, eine C₁₋₄-Aminoalkylgruppe, eine Phenylgruppe, die gegebenenfalls mit einer Nitrogruppe, einer Trifluormethylgruppe, einer Aminogruppe oder einer C₁₋₄-Alkylaminogruppe substituiert ist oder eine Benzylgruppe, die gegebenenfalls mit einem Halogenatom, einer C₁₋₄-Alkylgruppe, einer C₁₋₄-Alkoxygruppe, einer Methylendioxygruppe oder einer Aminogruppe substituiert ist, und mindestens ein substituiertes m-Aminophenolderivat der Formel (II) 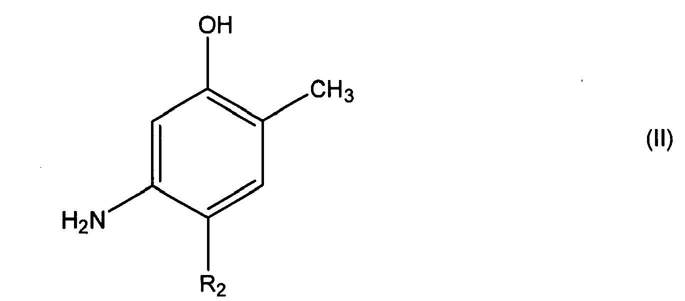 wobei R₂ steht für ein Halogenatom oder eine C₁₋₄-Alkoxygruppe, enthält.
